# EUROPÄISCHE PATENTSCHRIFT

(11) **EP 0 612 758 B1**
(45) Veröffentlichungstag und Bekanntmachung des Hinweises auf die Patenterteilung: **09.04.2003**
(21) Anmeldenummer: 94810092.0
(22) Anmeldetag: 17.02.1994
(51) Int. Cl.: C07F 17/02, B01J 31/28, C07B 31/00, C07B 53/00

(54) **Ferrocenyldiphosphine als Liganden für homogene Katalysatoren**
Ferrocenyl diphosphines as ligands for homogeneous catalysts
Ferrocenyl diphosphines comme ligands pour des catalyseurs homogènes

(30) Priorität: 26.02.1993 CH 59293
(43) Veröffentlichungstag der Anmeldung: 31.08.1994
(73) Patentinhaber: Syngenta Participations AG, 4058 Basel (CH)
(72) Erfinder: Spindler, Felix, Dr., CH-4656 Starrkirch-Wil (CH); Wirth-Tijani, Amina, Dr., F-68520 Burnhaupt-le-Haut (FR); Landert, Heidi, CH-4310 Rheinfelden (CH)
(74) Vertreter: Dannappel, Hans-Jochen, Dr.

(56) Entgegenhaltungen:
- EP-A- 0 564 406
- US-A- 5 128 478
- BULLETIN OF THE CHEMICAL SOCIETY OF JAPAN Bd. 53, Nr. 4 , 1980 , TOKYO JP Seiten 1138 - 1151 T. HAYASHI 'Asymmetric synthesis catalized by chiral ferrocenylphosphine-transition metal complexes.'

## Beschreibung

Die Erfindung betrifft 1-[2-Phosphinoferrocenyl]alkylidenphosphine in Form von Racematen und Stereoisomeren, ein Verfahren zu deren Herstellung, Iridium- und RhodiumKomplexe mit diesen Liganden und deren Verwendung als enantioselektive Hydrierkatalysatoren für die homogene Hydrierung von prochiralen ungesättigten Verbindungen.

T. Hayashi et al. beschreiben im Bull. Chem. Soc. Jpn., 53, Seiten 1136-1151 die Herstellung von einem chiralen Ferrocenylphosphin als Ligand für Übergangsmetallkomplexe zur asymmetrischen Synthese, nämlich [(R)-[(S)-2-(Diphenylphosphino)ferrocenyl]ethyl]diphenylphosphin. Eigene Untersuchungen ergaben, dass homogene Hydrierungen von prochiralen Verbindungen mit Rhodium-Komplexen, die diesen Liganden enthalten, nur niedrige optische Ausbeuten ergeben.

Es wurde nun gefunden, dass bei gleichen oder sogar kürzeren Reaktionszeiten die Enantioselektivität ganz wesentlich gesteigert werden kann, wenn die Substituenten in der 2-Phosphinogruppe nicht beide Phenyl darstellen.

Ein Gegenstand der Erfindung sind Verbindungen der Formel I, 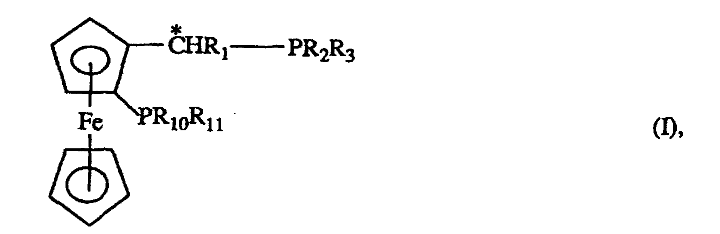 worin R₁ C₁-C₈-Alkyl, Phenyl oder mit 1 bis 3 C₁-C₄-Alkyl oder C₁-C₄-Alkoxy substituiertes Phenyl bedeutet; R₂ und R₃ unabhängig voneinander C₁-C₁₂-Alkyl, C₅-C₁₂-Cycloalkyl, Phenyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen; oder die Gruppe -PR₂R₃ einen Rest der Formel II 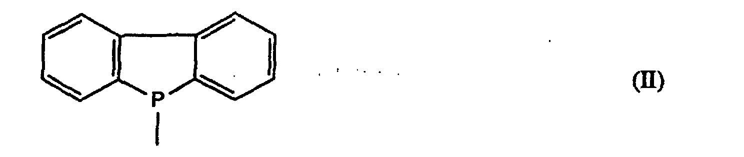 darstellt, und R₄, R₅ und R6 unabhängig voneinander für C₁-C₁₂-Alkyl oder Phenyl stehen, R₇ und R₈ H, C₁-C₁₂-Alkyl, Phenyl oder R₇ und R₈ zusammen Tetramethylen, Pentamethylen oder 3-Oxa-1,5-Pentylen darstellen, R₉ H oder C₁-C₄-Alkyl bedeutet, R₁₀ und R₁₁ gleich sind und C₁-C₁₂-Alkyl, C₅-C₁₂-Cycloalkyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen; oder R₁₀ und R₁₁ verschieden sind und C₁-C₁₂-Alkyl, C₅-C₁₂-Cycloalkyl, mit C₁-C₄-Alkyl oder C₁-C₄-Alkoxy substituiertes C₅-C₁₂-Cycloalkyl, Phenyl oder mit ein bis drei C₁-C₄-Alkyl, C₁-C₄-Alkoxy, -SiR₄R₅R₆, Halogen, -SO₃M, -CO₂M, -PO₃M₂, -NR₇R₈ oder -[^{⊕}NR₇R₈R₉]X^{⊖} substituiertes Phenyl bedeuten; oder die Gruppe -PR₁₀R₁₁ einen Rest der Formel II 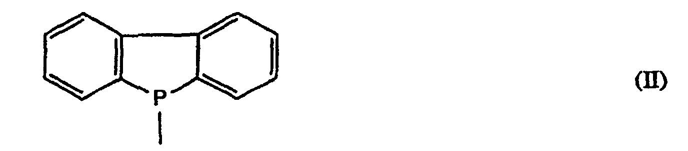 darstellt, M für H oder ein Alkalimetall steht, X^{⊖} das Anion einer einbasischen Säure ist, und * ein stereogenes C-Atom kennzeichnet, in Form ihrer Racemate und Diastereomeren oder Gemischen von Diastereomeren.

R₁ in der Bedeutung von Alkyl ist vorzugsweise linear. Es enthält bevorzugt 1 bis 4 C-Atome. Beispiele für dieses Alkyl sind Methyl, Ethyl, n- und i-Propyl, n-, i- und t-Butyl, Pentyl, Hexyl, Heptyl und Octyl. Bevorzugt sind Methyl und Ethyl und besonders bevorzugt ist Methyl.

R₁ enthält als substituiertes Phenyl bevorzugt 1 oder 2 Substituenten. Alkylsubstituenten können zum Beispiel Methyl, Ethyl, n- und i-Propyl, n-, i- und t-Butyl sein; bevorzugt sind Methyl und Ethyl. Alkoxysubstituenten können zum Beispiel Methoxy, Ethoxy, n-und i-Propoxy, n-, i- und t-Butoxy sein; bevorzugt sind Methoxy und Ethoxy. In einer bevorzugten Gruppe von Verbindungen der Formel I steht R₁ vorzugsweise für Phenyl oder mit 1 oder 2 C₁-C₄-Alkyl oder C₁-C₄-Alkoxy substituiertes Phenyl.

R₂, R₃, R₁₀ und R₁₁ in der Bedeutung von Alkyl können linear oder verzweigt sein und sie enthalten bevorzugt 1 bis 8 und besonders bevorzugt 1 bis 4 C-Atome. Beispiele für dieses Alkyl sind Methyl, Ethyl, n- und i-Propyl, n-, i- und t-Butyl, Pentyl, Hexyl, Heptyl, Octyl, Nonyl, Decyl, Undecyl und Dodecyl. Bevorzugt sind Methyl, Ethyl, n- und i-Propyl, n-, i-und t-Butyl. Wenn R₂, R₃ und/oder R₁₀ und R₁₁ gleich sind, bedeuten sie als Alkyl besonders bevorzugt i-Propyl oder t-Butyl.

R₂, R₃, R₁₀ und R₁₁ in der Bedeutung von Cycloalkyl enthalten bevorzugt 5 bis 8, besonders bevorzugt 5 oder 6 Ring-C-Atome. Beispiele für Cycloalkyl sind Cyclopentyl, Cyclohexyl, Cycloheptyl, Cyclooctyl, Cyclodecyl und Cyclododecyl. Bevorzugt sind Cyclopentyl und Cyclohexyl und besonders bevorzugt ist Cyclohexyl.

Das Cycloalkyl kann substituiert sein, zum Beispiel mit 1 bis 3 Alkyl- oder Alkoxy-Substituenten, Beispiele für solche Substituenten sind zuvor angegeben worden. Bevorzugt sind Methyl und Ethyl sowie Methoxy und Ethoxy. Beispiele für substituiertes Cycloalkyl sind Methyl- und Methoxycyclopentyl und -cyclohexyl.

R₂, R₃, R₁₀ und R₁₁ in der Bedeutung von substituiertem Phenyl enthält bevorzugt 1 oder 2 Substituenten. Sofern das Phenyl 2 oder 3 Substituenten enthält, können diese gleich oder verschieden sein.

Beispiele für die Substituenten Alkyl und Alkoxy sind zuvor angegeben worden; bevorzugte Alkyl- und Alkoxysubstituenten für Phenyl sind Methyl, Ethyl sowie Methoxy und Ethoxy.

Wenn der Phenyl-Substituent Halogen bedeutet, so handelt es sich bevorzugt um -F, -Cl und -Br.

R₄, R₅ und R₆ können lineares oder verzweigtes Alkyl sein, das bevorzugt 1 bis 8 und besonders bevorzugt 1 bis 4 C-Atome enthält. Beispiele für Alkyl sind zuvor angegeben worden. Bevorzugtes Alkyl ist Methyl, Ethyl, n-Propyl, n-Butyl und t-Butyl. Besonders bevorzugt steht der Substituent -SiR₄R₅R₆ für Trimethylsilyl.

Unter den sauren Phenyl-Substituenten -SO₃M, -CO₂M und -PO₃M ist die Gruppe -SO₃M bevorzugt. M steht bevorzugt für H, Li, Na und K.

R₇ und R₈ enthalten als Alkyl bevorzugt 1 bis 6, besonders bevorzugt 1 bis 4 C-Atome. Das Alkyl ist bevorzugt linear. Bevorzugte Beispiele sind Methyl, Ethyl, n-Propyl und n-Butyl. R₉ stellt als Alkyl bevorzugt Methyl dar.

X^{⊖} stellt als Anion einer einbasischen Säure bevorzugt Cl^{⊖}, Br^{⊖} oder das Anion einer Carbonsäure dar, zum Beispiel Formiat, Acetat, Trichloracetat oder Trifluoracetat dar.

Einige Beispiele für substituiertes Phenyl sind 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2-oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-SO₃H-, 2- oder 4-SO₃Na-, 2- oder 4-[^{⊕}NH₃Cl^{⊖}]-, 3,4,5-Trimethylphen-1-yl oder 2,4,6-Trimethylphen-1-yl.

Wenn R₂ und R₃ gleich sind, bedeuten R₂ und R₃ bevorzugt Phenyl, Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yL

Wenn R₂ und R₃ verschieden sind, bedeuten R₂ bevorzugt Phenyl und R₃ bevorzugt Cyclohexyl, 2- oder 4-Methylphen-1-yl, 2- oder 4-Methoxyphen-1-yl, 4-(Dimethylamino)-phen-1-yl, 3,5-Dimethyl-4-(dimethylamino)phen-1-yl, 3,5-Dimethyl-4-methoxyphen-1-yl oder 4-t-Butyl-phen-1-yl.

In einer bevorzugten Ausführungsform bedeuten R₂ und R₃ gleiche Reste und bedeuten Cyclohexyl oder Phenyl.

In einer besonders bevorzugten Ausführungsform bedeuten in Formel I R₁ Methyl und R₂ und R₃ je Cyclohexyl oder Phenyl.

Wenn R₁₀ und R₁₁ gleich sind, bedeuten R₁₀ und R₁₁ bevorzugt Cyclohexyl, t-Butyl, 2-oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl, besonders bevorzugt aber Cyclohexyl, 4-Methylphen-1-yl,3,5-Dimethylphen-1-yl und t-Butyl.

Wenn R₁₀ und R₁₁ verschieden sind, bedeuten R₁₀ bevorzugt Phenyl und R₁₁ bevorzugt Cyclohexyl, 2- oder 4-Methylphen-1-yl, 2- oder 4-Methoxyphen-1-yl, 4-(Dimethylamino)-phen-1-yl, 3,5-Dimethyl-4-(dimethylamino)phen-1-yl, 3,5-Dimethyl-4-methoxyphen-1-yl oder 4-t-Butyl-phen-1-yl.

Als besonders bevorzugte Verbindungen der Formel I sind zu nennen:
{(S)-1-[(R)-2-(Di-*para*-tolylphosphino)ferrocenyl]}ethyl-dicyclohexylphosphin,
{(R)-1-[(S)-2-(Dicyclohexylphosphino)ferrocenyl]}ethyl-dicyclohexylphosphin,
{(R)-1-[(S)-2-(Di-*tert*.-butylphosphino)ferrocenyl]}ethyl-diphenylphosphin,
{(R)-1-[(S)-2-(Dicyclohexylphosphino)ferrocenyl]}ethyl-diphenylphosphin und
{(R)-1-[(S)-2-(Di-(3,5-dimethylphenyl)phosphino)ferrocenyl]}ethyl-di-(3,5-dimethylphenyl)phosphin.

Die Verbindungen der Formel I werden hergestellt, indem man entweder eine Verbindung der Formel III 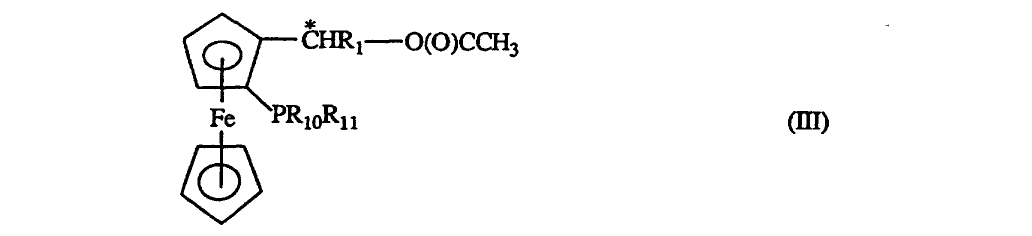 in Gegenwart eines inerten Lösungsmittels bei Raumtemperatur oder erhöhter Temperatur mit einem Phosphin der Formel IV

HPR₂R₃ (IV)

umsetzt; oder eine Verbindung der Formel VII 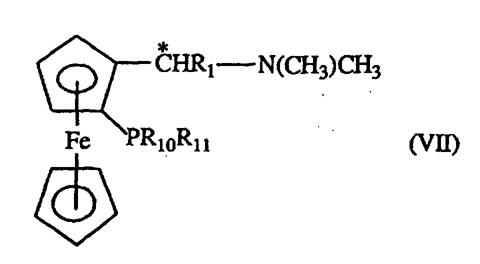 worin R₁, R₁₀ und R₁₁ die unter Formel I angegebenen Bedeutungen haben, in Gegenwart eines inerten Lösungsmittels bei Raumtemperatur oder erhöhter Temperatur mit einem Phosphin der Formel IV

HPR₂R₃ (IV)

umsetzt. Dieses Verfahren bildet einen weiteren Gegenstand der vorliegenden Erfindung.

Die Umsetzungen sind an sich bekannt und von T. Hayashi et al. im Bull. Chem. Soc. Jpn., 53, Seiten 1136-1151 beschrieben. Die Herstellung aller Stereoisomerer von Verbindungen der Formel III und VII ist dort ebenfalls beschrieben oder kann analog erfolgen. Die Phosphine der Formel IV sind bekannt oder nach bekannten Verfahren in analoger Weise herstellbar.

Die Reaktionstemperatur kann zum Beispiel 20 bis 150 °C, bevorzugt 40 bis 100 °C betragen. Als Lösungsmittel eignen sich polare protische und aprotische Lösungsmittel, die alleine oder in Mischung von zwei oder mehreren Lösungsmitteln verwendet werden können. Einige Beispiele für Lösungsmittel sind Alkanole wie zum Beispiel Methanol und Ethanol, und Carbonsäuren wie zum Beispiel Ameisensäure und Essigsäure.

Die Verbindungen der Formel I werden als Racemate, Gemische von Stereoisomeren oder als Stereoisomere erhalten, je nach dem, ob die Verbindungen der Formel III Racemate, Gemische von Stereoisomeren oder als Stereoisomere eingesetzt werden. Racemate und Gemische von Stereoisomeren können mittels bekannter Methoden in die Stereoisomeren getrennt werden, wobei im allgemeinen chromatographische Methoden bevorzugt werden.

Die Isolierung und Reinigung der Verbindungen der Formel I erfolgt nach an sich bekannten Methoden, zum Beispiel Destillation, Extraktion, Kristallisation und/oder chromatographischen Methoden.

Die erfindungsgemässen Verbindungen der Formel I eignen sich als Liganden für Rhodium- und Iridiumkomplexe. Ein weiterer Gegenstand der Erfindung sind Komplexe der Formeln V und VI,

[X₁M₁YZ] (V),

[X₁M₁Y]^{⊕}A₁^{⊖} (VI),

worin X₁ für zwei C₂-C₁₂-Olefine oder ein C₅-C₁₂-Dien steht, Z für Cl, Br oder I steht, A₁^{⊖} das Anion einer Sauerstoffsäure oder Komplexsäure bedeutet, M₁ für Rh oder Ir steht und Y ein erfindungsgemässes Diphosphin der Formel I darstellt. Die Komplexe der Formel VI sind bevorzugt.

Für die Diphosphine der Formel I gelten die gleichen zuvor angeführten Bevorzugungen und beispielhaften Ausführungen. X₁ in der Bedeutung als Olefin enthält bevorzugt 2 bis 6 und besonders bevorzugt 2 bis 4 C-Atome. Besonders bevorzugt ist Ethylen; weitere Beispiele sind Propen und 1-Buten. X₁ enthält als Dien bevorzugt 5 bis 8 C-Atome, wobei es sich um ein offenkettiges oder mono- oder bicyclisches Dien handeln kann. Die beiden Olefingruppen des Diens sind bevorzugt durch ein oder zwei CH₂-Gruppen verbunden. Beispiele sind 1,3-Pentadien, Cyclopentadien, 1,5-Hexadien, 1,4-Cyclohexadien, 1,4- oder 1,5-Heptadien, 1,4- oder 1,5-Cycloheptadien, 1,4- oder 1,5-Octadien, 1,4- oder 1,5-Cyclooctadien, Norbornadien. Bevorzugt stellt X₁ zwei Ethylen, 1,5-Hexadien, 1,5-Cyclooctadien oder Norbornadien dar.

In Formel V steht Z bevorzugt für Cl oder Br. Beispiele für A₁^{⊖} in Formel VI sind ClO₄^{⊖}, FSO₃^{⊖}, CH₃SO₃^{⊖}, CF₃SO₃^{⊖}, BF₄^{⊖}, PF₆^{⊖}, SbCl₆^{⊖}, AsF₆^{⊖} und SbF₆^{⊖}. Bevorzugt ist A₁^{⊖} ClO₄^{⊖}, CF₃SO₃^{⊖}, BF₄^{⊖}, PF₆^{⊖} und SbF₆^{⊖}.

Die erfindungsgemässen Komplexe werden in an sich bekannter Weise durch die Umsetzung von äquimolaren Mengen einer Verbindung der Formel I mit einem Metallkomplex der Formeln [M₁(X₁)Z]₂ oder M₁(X₁)₂^{⊕}A₁^{⊖} erhalten, worin M₁, X₁, Z und A₁^{⊖} die zuvor angegebenen Bedeutungen haben. Die Metallkomplexe sind bekannt, siehe zum Beispiel EP-A-0 302 021 uns US-A-5 011 995.

Die Umsetzung wird vorteilhaft unter Inertgasatmosphäre, zum Beispiel Argon, und zweckmässig bei Temperaturen von 0 bis 40 °C, bevorzugt bei Raumtemperatur vorgenommen. Vorteilhaft wird ein Lösungsmittel oder Gemisch von Lösungsmitteln mitverwendet, zum Beispiel Kohlenwasserstoffe (Benzol, Toluol, Xylol), halogenierte Kohlenwasserstoffe (Methylenchlorid, Chloroform, Chlorbenzol), Alkohole (Methanol, Ethanol, Ethylenglykolmonomethylether), und Ether (Diethylether, Dibutylether, Ethylenglykoldimethylether, Tetrahydrofuran, Dioxan) oder Gemische davon. Die erfindungsgemässen Komplexe können nach üblichen Methoden isoliert und gereinigt werden, oder sie können vor einer Hydrierung in situ hergestellt und dann in gelöster Form direkt als Hydrierkatalysator eingesetzt werden.

Die erfindungsgemässen Komplexe eignen sich hervorragend als homogene Katalysatoren zur enantioselektiven Hydrierung von prochiralen Verbindungen mit Kohlenstoff- und Kohlenstoff-/Heteroatomdoppelbindungen, zum Beispiel Verbindungen, die eine der Gruppen C=C, C=N, C=O, C=C-N oder C=C-O enthalten [siehe zum Beispiel K. E. König, The Applicability of Asymmetric Homogeneous Catalysis, in James D. Morrison (ed.), Asymmetric Synthesis, Vol. 5, Academic Press, 1985]. Beispiele für solche Verbindungen sind prochirale Olefine, Enamine, Imine und Ketone. Es werden überraschend hohe Ausbeuten, im allgemeinen sogar ein quantitativer chemischer Umsatz, in kurzen Reaktionszeiten erzielt. Besonders überraschend sind die sehr hohen optischen Ausbeuten, die mit den erfindungsgemässen Komplexen erreicht werden. Der Enantiomerenüberschuss (ee) kann über 90 % betragen. Es können hierbei Racemate, Gemische von Stereoisomeren oder Stereoisomere der Komplexe der Formeln V und VI verwendet werden, wobei Gemische von Stereoisomeren oder Stereoisomere bevorzugt sind.

Ein weiterer Gegenstand der Erfindung ist die Verwendung der erfindungsgemässen Komplexe der Formeln V und VI als homogene Katalysatoren zur asymmetrischen Hydrierung von prochiralen Verbindungen mit Kohlenstoff- oder Kohlenstoff-/Heteroatomdoppelbindungen, besonders mit Gruppen C=C, C=N, C=N-N, C=O, C=C-N oder C=C-O. Bevorzugt ist die Verwendung zur Hydrierung von unsymmetrischen Kohlenstoffdoppelbindungen, Ketiminen und Ketonen. Der erfindungsgemäss als Iridium-Komplex ausgebildete Katalysator der Formeln V und VI wird auch bevorzugt zur Hydrierung von prochiralen N-Arylketiminen zu optisch aktiven sekundären Aminen verwendet Der erfindungsgemäss als Rhodium-Komplex ausgebildete Katalysator der Formeln V und VI wird bevorzugt zur Hydrierung von Kohlenstoffdoppelbindungen, zum Beispiel prochiralen Kohlenstoffdoppelbindungen verwendet.

Ein anderer Gegenstand der Erfindung ist ein Verfahren zur asymmetrischen Hydrierung von prochiralen Verbindungen mit Kohlenstoff- oder Kohlenstoff-/Heteroatomdoppelbindungen unter homogenen Reaktionsbedingungen, das dadurch gekennzeichnet ist, dass man die Verbindungen bei einer Temperatur von -20 bis 80 °C und einem Wasserstoffdruck von 10⁴ bis 10⁷ Pa in Gegenwart katalytischer Mengen eines Komplexes der Formeln V oder VI hydriert.

Bevorzugte prochirale Verbindungen sind zuvor erwähnt worden. Unsymmetrische Ketimine und Ketone sind bekannt. In Frage kommende N-Arylketimine sind zum Beispiel in der EP-A-0 256 982 beschrieben. N-aliphatische Ketimine sind zum Beispiel in der EP-A-0 301 457 beschrieben. Solche Imine können aus den entsprechenden unsymmetrische Ketonen hergestellt werden, die bekannt und teilweise käuflich oder nach bekannten Verfahren herstellbar sind. Geeignete substituierte Alkene sind in der zuvor erwähnten Publikation von K. E. König beschrieben.

Das Verfahren wird bevorzugt bei einer Temperatur von -10 bis 50 °C und bevorzugt bei einem Wasserstoffdruck von 1·10⁵ bis 6·10⁶ Pa durchgeführt

Die Menge des Katalysators wird bevorzugt so gewählt, dass das Molverhältnis der zu hydrierenden Verbindung (Substrat) zu dem Komplex der Formeln V oder VI bevorzugt 100000 bis 20, besonders bevorzugt 20000 bis 20, insbesondere bevorzugt 5000 bis 40, und ganz besonders bevorzugt 1000 bis 50 beträgt.

Eine bevorzugte Verfahrensdurchführung ist dadurch gekennzeichnet, dass man zusätzlich ein Ammonium- oder Alkalimetallchlorid, -bromid oder -iodid mitverwendet, besonders bei der Verwendung erfindungsgemässer Iridium-Katalysatoren. Die Menge kann beispielsweise 0,1 bis 100, bevorzugt 1 bis 50 und besonders bevorzugt 2 bis 20 Aequivalente betragen, bezogen auf den Komplex der Formeln V oder VI. Der Zusatz von Iodiden ist bevorzugt. Ammonium ist bevorzugt Tetraalkylammonium mit 1 bis 6 C-Atomen in den Alkylgruppen, und als Alkalimetall ist Lithium, Natrium und Kalium bevorzugt

Die Hydrierung kann ohne oder in Gegenwart von Lösungsmitteln durchgeführt werden. Geeignete Lösungsmittel, die alleine oder als Mischung von Lösungsmitteln eingesetzt werden können, sind zum Beispiel: Aliphatische und aromatische Kohlenwasserstoffe (Pentan, Hexan, Cyclohexan, Methylcyclohexan, Benzol, Toluol, Xylol), Alkohole wie z.B. Methanol, Ethanol, Propanol und Butanol; Ether wie z.B. Diethylether, Diethylenglykoldimethylether, Ethylenglykoldimethylether, Tetrahydrofuran und Dioxan; Halogenkohlenwasserstoffe wie z.B. Methylenchlorid, Chloroform, 1,1,2,2-Tetrachlorethan und Chlorbenzol; Ester und Lactone wie z.B. Essigsäureethylester, Butyrolacton oder Valerolacton; Carbonsäureamide und Lactame wie z.B. Dimethylformamid, Dimethylacetamid und N-Methylpyrrolidon. Bevorzugte Mischungen sind solche aus Alkoholen und aromatischen Kohlenwasserstoffen, zum Beispiel Methanol/Benzol oder Methanol/Toluol. Bevorzugt wird als Lösungsmittel Methanol alleine oder im Gemisch mit Benzol oder Toluol verwendet.

In einer besonders bevorzugten Ausgestaltung des erfindungsgemässen Verfahrens werden N-2,6-Dialkylphen-4-ylketimine, zum Beispiel N-2,6-Dimethyl- oder N-2-Methyl-6-ethylphen-4-yl-methoxyacetonimin hydriert.

Mit dem erfindungsgemässen Hydrierverfahren können optisch reine Verbindungen erhalten werden, die wertvolle Zwischenprodukte zur Herstellung biologisch aktiver Wirkstoffe besonders im Bereich der Pharmazeutika und Agrarchemikalien darstellen. So können zum Beispiel aus sekundären Aminen, besonders N-Carbalkoxymethylaminen, 5-Imidazolcarbonsäurederivate hergestellt werden, die eine herbizide Wirkung haben und zur Bekämpfung von Unkräutern verwendet werden können (EP-A-0 207 563). Die optisch reinen α-Aminocarbonsäureester sind für Peptidsynthesen geeignet. Aus ungesättigten Aminocarbonsäuren können optisch reine Aminocarbonsäuren erhalten werden, die wertvolle Synthesebausteine darstellen.

Die nachfolgenden Beispiele erläutern die Erfindung näher. Der chemische Umsatz wird gaschromatographisch bestimmt [Säule DB 17/30 W (15 m), Hersteller: JCW Scientific INC., USA, Temperaturprogramm: 60°C/ 1 min bis 220°C, ΔT: 10°·min⁻¹]. Die Bestimmung der optischen Ausbeute Enantiomerenüberschuss, ee) erfolgt entweder gaschromatographisch [Säule Chirasil-Val, 50 m, Hersteller: Alltech, USA, T: 150°C, isotherm), mittels HPLC (Säule Chiracel OD) oder mittels ¹H-NMR-Spektroskopie unter Verwendung von Shift-Reagenzien.

### A) Herstellungsbeispiele

### Beispiel A1: [(S)-1-[(R)-2-(Di-para-tolylphosphino)ferrocenyl]}ethyl-dicyclohexylphosphin (A).

In einen 25 ml Schlenk-Kolben werden unter Argon-Atmosphäre nacheinander 1,2 g (2,56 mmol) N-{(S)-1-[(R)-2-(Di-*para*-tolylphosphino)ferrocenyl]}ethyl-dimethylamin [hergestellt aus N-[(S)-1-Ferrocenyl]ethyl-dimethylamin und Di-*para*-tolylphosphinchlorid gemäss Bull. Chem. Soc. Jpn., **53**, 1138 (1980)], 15 ml Essigsäure und 0,62 ml (3,07 mmol) Dicyclohexylphosphin eingetragen und anschliessend während 25 Minuten unter Rühren bei 100°C. erhitzt. Dann wird das Rohprodukt aus Wasser/Toluol extrahiert. Die organische Phase wird mit Natriumsulfat getrocknet und das Lösungsmittel am Rotationsverdampfer entfernt. Nach einer chromatographischen Reinigung über Kieselgel (Lösungsmittel: Diethylether) und der anschliessenden Umkristallisation des Rohprodukts aus heissem Ethanol erhält man 1,4 A (Ausbeute: 80%) als orange-gelbe kristalline Substanz. [α]²²_{D}:+342° (c=0,41, CHCl₃); ³¹P-NMR (CDCl₃): 15,5 (d,J=27), -27,8 (d,J=27).

### Beispiel A2: {(R)-1-[(S)-2-(Dicyclohexylphosphino)ferrocenyl]}ethyl-dicyclohexylphosphin (B).

Es wird analog Beispiel A1 verfahren, aber 0,262 g (0,579 mmol) N-{(R)-1-[(S)-2-(Dicyclohexylphosphino)ferrocenyl]}ethyl-dimethylamin, 4 ml Essigsäure und 0,14 ml (0,695 mmol) Dicyclohexylphosphin verwendet. Die Ausbeute beträgt 0,23 g **B** (Ausbeute: 67%) als orange kristalline Substanz. ³¹P-NMR (CDCl₃): 12,6 (d,J=6), -12,7 (d,J=6).

### Beispiel A3: {(R)-1-[(S)-2-(Di-tert.-butylphosphino)ferrocenyl]}ethyl-diphenylphosphin (C).

Es wird analog Beispiel A1 verfahren, aber 0,546 g (1,36 mmol) N-{(R)-1-[(S)-2-(Ditert.-butylphosphino)ferrocenyl] }ethyl-dimethylamin, 5 ml Essigsäure und 0,28 ml (1,63 mmol) Diphenylphosphin verwendet. Die Ausbeute beträgt 0,37 g **C** (Ausbeute: 50%) als orange kristalline Substanz. ³¹P-NMR (CDCl₃): 13,5 (d,J=10), -1,5 (d,J=10).

### Beispiel A4: {(R)-1-[(S)-2-(Dicyclohexylphosphino)ferrocenyl]}ethyl-diphenylphosphin (D).

Es wird analog Beispiel A1 verfahren, aber 0,24 g (0,53 mmol) N-{(R)-1-[(S)-2-(Dicyclohexylphosphino)ferrocenyl]}ethyl-dimethylamin, 4 ml Essigsäure und 0,11 ml (0,64 mmol) Diphenylphosphin verwendet. Die Ausbeute beträgt 0,12 g **D** (Ausbeute: 38 %) als orange kristalline Substanz. ³¹P-NMR (CDCl₃): 4,6 (d,J=6), -13,4 (d,J=6).

### Beispiel A5: {(R)-1-[(S)-2-(Di-(3,5-dimethylphenyl)phosphino)ferrocenyl]}ethyl-di(3,5-dimethylphenyl)phosphin (E).

Es wird analog Beispiel A1 verfahren und die Reaktionsbedingungen wie folgt abgewandelt:
1,0 g (2,0 mmol) {(R)-1-[(S)-2-(Di-(3,5-dimethylphenyl)phosphino)ferrocenyl]}ethyl-dimethylamin), 0,53 g (2,2 mmol) Di-(3,5-dimethylphenyl)phosphin und 26 ml Essigsäure. Die Ausbeute beträgt 0,69 mg **E** (49%) als orange kristalline Substanz. ³¹P-NMR (CDCl₃): 7,9 (d, J=20), -24,7 (d, J=20).

### Beispiel A6: {(R)-1-[(S)-2-(Dicyclohexylphosphino)ferrocenyl]}ethyl-di-tert.-butylphosphin (F).

Es wird analog Beispiel A1 verfahren und die Reaktionsbedingungen wie folgt abgewandelt:
0,91 g (2,0 mmol) {(R)-1-[(S)-2-(Dicyclohexylphosphino)ferrocenyl]}ethyl-dimethylamin), 0,34 g (2,3 mmol) Di-*tert*.-butylphosphin und 12 ml Essigsäure. Die Ausbeute beträgt 330 mg **F** (30%) als orange, geschäumte Substanz; ³¹P-NMR (CDCl₃): 46,6 (d, J=16), -15,4 (d, J=16).

### Beispiel A7: {(R)-1-[(S)-2-(Di-(2-naphtyl)phosphino)ferrocenyl]}ethyl-diphenylphosphin (G).

Es wird analog Beispiel A1 verfahren und die Reaktionsbedingungen wie folgt abgewandelt:
380 mg (0,7 mmol) {(R)-1-[(S)-2-(Di-(2-naphtyl)phosphino)ferrocenyl]}ethyl-dimethylamin), 134 µl (0,77 mmol) Diphenylphosphin und 6 ml Essigsäure. Die Ausbeute beträgt 203 mg **G** (42,5%) als orange kristalline Substanz; ³¹P-NMR (CDCl₃): 7,0 (d, J=22), -23,3 (d, J=22).

### Beispiel A8: {(R)-1-[(S)-2-(Di-(3,5-dimethylphenyl)phosphino)ferrocenyl]}ethyl-diphenylphosphin (H).

Es wird analog Beispiel A1 verfahren und die Reaktionsbedingungen wie folgt abgewandelt:
1,0 g (2,0 mmol) ((R)-1-[(S)-2-(Di-(3,5-dimethylphenyl)phosphino)ferrocenyl] }ethyl-dimethylamin), 0,4 ml (2,3 mmol) Diphenylphosphin und 15 ml Essigsäure. Die Ausbeute beträgt 804 mg **H** (63%) als orange kristalline Substanz; ³¹P-NMR (CDCl₃): 5,8 (d, J=20), -25,3 (d, J=20).

### Beispiel A9: {(R)-1-[(S)-2-(Di-(2-naphtyl)phosphino)ferrocenyl]}ethyl-di(3,5-dimethylphenyl)phosphin (I).

Es wird analog Beispiel A1 verfahren und die Reaktionsbedingungen wie folgt abgewandelt:
1,08 g (2,0 mmol) {(R)-1-[(S)-2-(Di-(2-naphtyl)phosphino)ferrocenyl]}ethyl-dimethylamin), 0,53 g (2,2 mmol) Di-(3,5-dimethylphenyl)phosphin und 15 ml Essigsäure. Die Ausbeute beträgt 1,07 g **I** (72,3%) als orange kristalline Substanz; ³¹P-NMR (CDCl₃): 8,1 (d, J=20), -23,9 (d, J=20).

### B) Anwendungsbeispiele

### Beispiel B1: Herstellung von N-Acetylalaninmethylester

In einen 200 ml Glasreaktor wird unter Argonatmosphäre mittels einer Stahlkapillare eine Katalysatorlösung (unter Argon hergestellt) transferiert, die aus 12,8 mg (0,034 mmol) [Rh(Norbornadien)₂]BF₄, 22,6 mg (0,036 mmol) A und 5 ml Methanol besteht. Anschliessend wird analog eine Lösung, bestehend aus 750 mg (3,42 mmol) Z-Methyl-2-acetamidocinnamat (Substrat) und 5 ml Methanol zugegeben. Das Molverhältnis Substrat/Katalysator beträgt 100. Dann werden in drei Spülzyklen 0,1 MPa Wasserstoff aufgepresst und der Wasserstoffdruck auf 0,108 MPa eingestellt. Die Reaktionsmischung wird bei 25°C 30 Minuten lang gerührt und darauf in einen Kolben übergeführt und das Lösungsmittel am Rotationsverdampfer entfernt. Bei einem chemischen Umsatz von 100% erhält man N-Acetylalaninmethylester mit einem Enantiomerenüberschuss (ee) von 91,4% (S).

### Beispiel B2: Herstellung von N-Acetylalaninmethylester

Es wird analog Beispiel B1 verfahren und die Reaktionsbedingungen wie folgt abgewandelt 21,7 mg (0,036 mmol) **B.** Der Umsatz beträgt 100%, ee: 75%(R).

### Beispiel B3: Herstellung von N-(2'-Methyl-6-ethyl-phen-1'-yl)-N-(1-methoxymethyl)-ethylamin

Es werden nacheinander 5 ml (24 mmol) (2'-Methyl-6-ethyl-phen-1'-yl)-N-(1-methoxymethyl)eth-1-ylidenamin, 10,2 mg (0,015 mmol) [Ir(1,5-Cyclooctadien)Cl]₂, 23,2 mg (0,033 mmol) E und 50 mg Tetrabutylammoniumiodid in einen 50 ml Stahlautoklaven eingetragen. Das Verhältnis Imin/Katalysator beträgt 800. Der Autoklav wird verschlossen und anschliessend in drei Spülzyklen unter Inertgas (Argon) gesetzt. 20 ml i-Propanol werden mittels einer Stahlkapillare unter Luftausschluss in den Autoklaven transferiert. In drei weiteren Zyklen (2 MPa, Normaldruck) wird das Argon durch Wasserstoff verdrängt. Es werden 2,5 MPa Wasserstoff aufgepresst. Nach einer Reaktionsdauer von 18 Std. bei Raumtemperatur wird die Reaktion abgebrochen. Der Umsatz beträgt 100%, die Enantiomerenreinheit 81,6% (S).

### Beispiel B4: Herstellung von N-(2'-Methyl-6-ethyl-phen-1'-yl)-N-(1-methoxymethyl)-ethylamin

Es wird analog Beispiel B3 verfahren und die Reaktionsbedingungen wie folgt abgewandelt:
**G** 22,8 mg (0,033 mmol), Reaktionszeit 18 Stunden. Der Umsatz ist 62%, ee: 75% (S).

### Beispiel B5: Herstellung von N-(2'-Methyl-6-ethyl-phen-1'-yl)-N-(1-methoxymethyl)-ethylamin

Es wird analog Beispiel B3 verfahren und die Reaktionsbedingungen wie folgt abgewandelt:
**I** 24,6 mg (0,033 mmol), Reaktionszeit 18 Stunden. Der Umsatz ist 77%, ee: 80,4% (S).

### Beispiel B6: Herstellung von Methyl-3-hydroxybutyrat

Alle Manipulationen werden unter einer Argonatmosphäre durchgeführt. Zu einer Lösung von 5,1 mg (0,011 mmol) [Rh(Norbornadien)Cl]₂ in 10 ml Methanol werden 14,4 mg (0,023 mmol) **B** eingetragen. Separat werden 0,51 g (4,4 mmol) Methylacetylacetat in 5 ml Methanol gelöst. Die Substrat- und die Katalysatorlösung werden nacheinander mittels einer Stahlkapillare in einen unter Argon stehenden 50 ml-Stahlautoklaven transferiert. In drei Zyklen (2 MPa, Normaldruck) wird das Inertgas durch Wasserstoff verdrängt. Anschliessend werden 2,5 MPa Wasserstoff aufgepresst. Nach einer Reaktionsdauer von 20 Std. bei Raumtemperatur wird die Reaktion abgebrochen. Der Umsatz beträgt 100%, die Enantiomerenreinheit 94,5% (S).

### Beispiel B7: Herstellung von Methyl-3-hydroxybutyrat

Es wird analog Beispiel B6 verfahren und die Reaktionsbedingungen wie folgt abgewandelt:
14,9 mg (0,024 mmol) **A**. Nach einer Reaktionsdauer von 24 Stunden beträgt der Umsatz 100%, die Enantiomerenreinheit 84,4% (S).

### Beispiel B8: Herstellung von Methyl-mandelat

Es wird analog Beispiel B6 verfahren und die Reaktionsbedingungen wie folgt abgewandelt:
0,268 g (1,63 mmol) Methyl-phenylglyoxylat, 3,9 mg (0,0084 mmol) [Rh(Norbornadien)Cl]₂, 9,4 mg (0,018 mmol) **C**, 10 ml Methanol. Der Wasserstoffpartialdruck beträgt 4 MPa, die Reaktionstemperatur 25°C. Nach einer Reaktionsdauer von 21 Stunden beträgt der Umsatz 74%, die optische Ausbeute 52%.

### Beispiel B9: Herstellung von N-Acetylalaninmethylester

Es wird analog Beispiel B1 verfahren und die Reaktionsbedingungen wie folgt abgewandelt:
[Rh(Norbornadien)₂]BF₄ 6,4 mg (0,017 mmol), **E** 13,1 mg (0,019 mmol), Z-Methyl-2-acetamidocinnamat (Substrat) 0,75 g (3,42 mmol), Methanol 15 ml, Wasserstoffpartialdruck 2 MPa, Temperatur 40°C., Reaktionszeit 2 Stunden. Der Umsatz ist 100%, ee 70% (S).

## Patentansprüche

1. Verbindungen der Formel I, 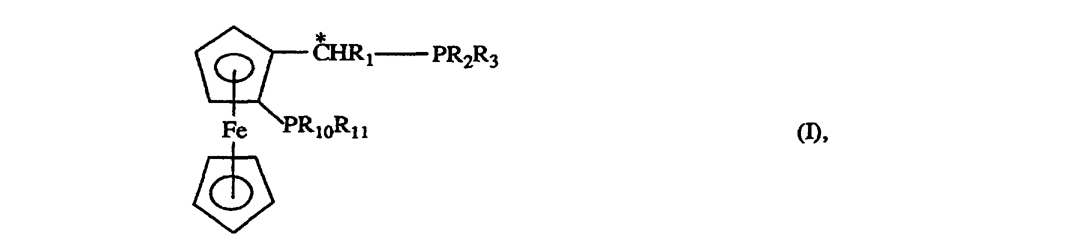 worin R₁ C₁-C₈-Alkyl, Phenyl oder mit 1 bis 3 C₁-C₄-Alkyl oder C₁-C₄-Alkoxy substituiertes Phenyl bedeutet; R₂ und R₃ unabhängig voneinander C₁-C₁₂-Alkyl, C₅-C₁₂-Cycloalkyl, Phenyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen; oder die Gruppe -PR₂R₃ einen Rest der Formel II 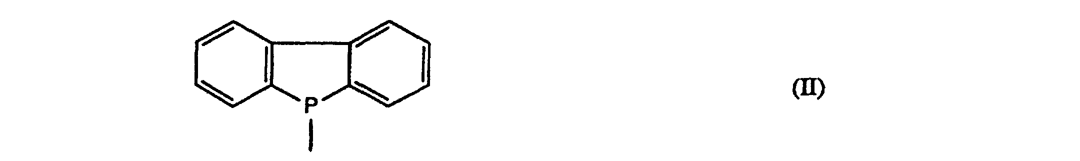 darstellt, und R₄, R₅ und R₆ unabhängig voneinander für C₁-C₁₂-Alkyl oder Phenyl stehen, R₇ und R₈ H, C₁-C₁₂-Alkyl, Phenyl oder R₇ und R₈ zusammen Tetramethylen, Pentamethylen oder 3-Oxa-1,5-Pentylen darstellen, R₉ H oder C₁-C₄-Alkyl bedeutet, R₁₀ und R₁₁ gleich sind und C₁-C₁₂-Alkyl, C₅-C₁₂-Cycloalkyl, mit C₁-C₄-Alkyl oder C₁-C₄-Alkoxy substituiertes C₅-C₁₂-Cycloalkyl oder mit ein bis drei C₁-C₄-Alkyl, C₁-C₄-Alkoxy, -SiR₄R₅R₆, Halogen, -SO₃M, -CO₂M, -PO₃M, -NR₇R₈ oder -[^{⊕}NR₇R₈R₉]X^{⊖} substituiertes Phenyl darstellen; oder R₁₀ und R₁₁ verschieden sind und C₁-C₁₂-Alkyl, C₅-C₁₂-Cycloalkyl, mit C₁-C₄-Alkyl oder C₁-C₄-Alkoxy substituiertes C₅-C₁₂-Cycloalkyl, Phenyl oder mit ein bis drei C₁-C₄-Alkyl, C₁-C₄-Alkoxy, -SiR₄R₅R₆, Halogen, -SO₃M, -CO₂M, -PO₃M₂, -NR₇R₈ oder -[^{⊕}NR₇R₈R₉]X^{⊖} substituiertes Phenyl bedeuten; oder die Gruppe -PR₁₀R₁₁ einen Rest der Formel II 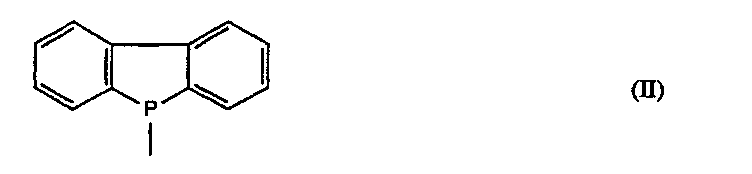 darstellt, M für H oder ein Alkalimetall steht, X^{⊖} das Anion einer einbasischen Säure ist, und * ein stereogenes C-Atom kennzeichnet, in Form ihrer Racemate und Diastereomeren oder Gemischen von Diastereomeren.

2. Verbindungen der Formel I gemäss Anspruch 1, **dadurch gekennzeichnet, dass** R₁ als Alkyl linear ist.

3. Verbindungen der Formel I gemäss Anspruch 2, **dadurch gekennzeichnet, dass** R₁ C₁-C₄-Alkyl darstellt.

4. Verbindungen der Formel I gemäss Anspruch 3, **dadurch gekennzeichnet, dass** R₁ für Methyl oder Ethyl steht.

5. Verbindungen der Formel I gemäss Anspruch 1, **dadurch gekennzeichnet, dass** R₁ Phenyl oder mit 1 oder 2 C₁-C₄-Alkyl oder C₁-C₄-Alkoxy substituiertes Phenyl darstellt.

6. Verbindungen der Formel I gemäss Anspruch 1, **dadurch gekennzeichnet, dass** R₂ und R₃ C₁-C₈-Alkyl darstellen.

7. Verbindungen der Formel I gemäss Anspruch 6, **dadurch gekennzeichnet, dass** R₂ und R₃ gleich sind und für i-Propyl oder t-Butyl stehen.

8. Verbindungen der Formel I gemäss Anspruch 6, **dadurch gekennzeichnet, dass** R₁₀ und R₁₁ gleich sind und für i-Propyl oder t-Butyl stehen.

9. Verbindungen der Formel I gemäss Anspruch 1, **dadurch gekennzeichnet, dass** R₂, R₃, R₁₀ und R₁₁ als Cycloalkyl 5 bis 8 Ring-C-Atome enthält.

10. Verbindungen der Formel I gemäss Anspruch 9, **dadurch gekennzeichnet, dass** R₂, R₃, R₁₀ und R₁₁ als Cycloalkyl Cyclopentyl oder Cyclohexyl darstellt.

11. Verbindungen der Formel I gemäss Anspruch 1, **dadurch gekennzeichnet, dass** R₂, R₃, R₁₀ und R₁₁ als substituiertes Phenyl 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2- oder 4-SO₃H-, 2- oder 4-SO₃Na-, 2- oder 4-[^{⊕}NH₃Cl^{⊖}]-, 2,4,6-Trimethylphen-1-yl oder 3,4,5-Trimethylphen-1-yl darstellen.

12. Verbindungen der Formel I gemäss Anspruch 1, **dadurch gekennzeichnet, dass** R₂ und R₃ gleich sind und Phenyl, Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl bedeuten.

13. Verbindungen der Formel I gemäss Anspruch 1, **dadurch gekennzeichnet, dass** R₂ und R₃ verschieden sind und R₂ Phenyl und R₃ Cyclohexyl, 2- oder 4-Methylphen-1-yl, 2-oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

14. Verbindungen der Formel I gemäss Anspruch 1, **dadurch gekennzeichnet, dass** R₂ und R₃ gleiche Reste darstellen und Cyclohexyl oder Phenyl bedeuten.

15. Verbindungen der Formel I gemäss Anspruch 1, **dadurch gekennzeichnet, dass** R₁₀ und R₁₁ gleiche Reste darstellen und Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl, 3,5-Dimethylphen-1-yl oder 3,5-Dimethyl-4-methoxyphen-1-yl bedeuten.

16. Verbindungen der Formel I gemäss Anspruch 1, **dadurch gekennzeichnet, dass** R₁₀ Phenyl und R₁₁ Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

17. Verbindungen der Formel I gemäss Anspruch 1, **dadurch gekennzeichnet, dass** R₁ Methyl und R₂ und R₃ je Cyclohexyl oder Phenyl bedeuten.

18. Verfahren zur Herstellung von Verbindungen der der Formel I gemäss Anspruch 1, **dadurch gekennzeichnet, dass** man
entweder eine Verbindung der Formel III 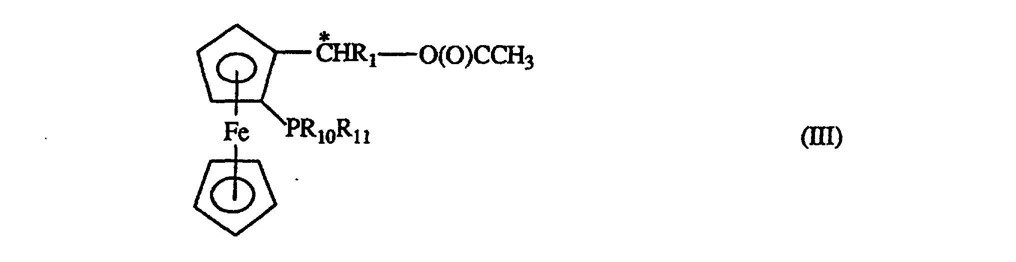 in Gegenwart eines inerten Lösungsmittels bei Raumtemperatur oder erhöhter Temperatur mit einem Phosphin der Formel IV
HPR₂R₃ (IV)
umsetzt; oder eine Verbindung der Formel VII 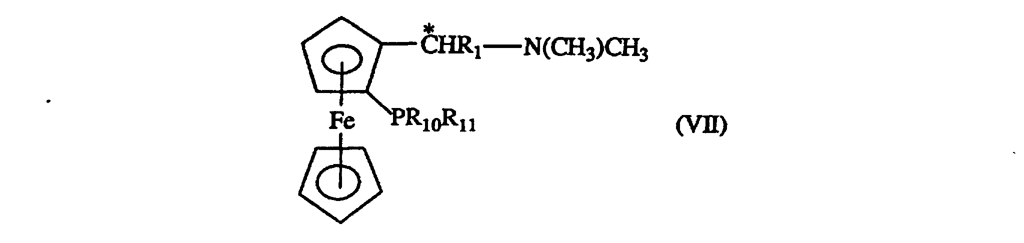 worin R₁, R₁₀ und R₁₁ die unter Formel I angegebenen Bedeutungen haben, in Gegenwart eines inerten Lösungsmittels bei Raumtemperatur oder erhöhter Temperatur mit einem Phosphin der Formel IV
HPR₂R₃ (IV)
umsetzt, wobei R₁, R₂, R₃, R₁₀ und R₁₁ die in Anspruch 1 angegebenen Bedeutungen haben.

19. Komplexe der Formeln V und VI,
[X₁M₁YZ] (V),
[X₁M₁Y]^{⊕}A₁^{⊖} (VI),
worin X₁ für zwei C₂-C₁₂-Olefine oder ein C₅-C₁₂-Dien steht, Z für Cl, Br oder I steht, A₁^{⊖} das Anion einer Sauerstoffsäure oder Komplexsäure bedeutet, M₁ für Rh oder Ir steht und Y eine Verbindung der Formel I gemäss Anspruch 1 darstellt.

20. Komplexe gemäss Anspruch 19, **dadurch gekennzeichnet, dass** es sich um solche der Formel VI handelt.

21. Komplexe gemäss Anspruch 19, **dadurch gekennzeichnet, dass** X₁ zwei Ethylen, 1,5-Hexadien, 1,5-Cyclooctadien oder Norbornadien darstellen.

22. Komplexe gemäss Anspruch 19, **dadurch gekennzeichnet, dass** Z für Cl oder Br steht.

23. Komplexe gemäss Anspruch 19, **dadurch gekennzeichnet, dass** Z für ClO₄^{⊖}, FSO₃^{⊖}, CH₃SO₃^{⊖}, CF₃SO₃^{⊖}, BF₄^{⊖}, PF₆^{⊖}, SbCl₆^{⊖}, AsF₆^{⊖} und SbF₆^{⊖} steht.

24. Komplexe gemäss Anspruch 19, **dadurch gekennzeichnet, dass** R₁ als Alkyl linear ist.

25. Komplexe gemäss Anspruch 24, **dadurch gekennzeichnet, dass** R₁ C₁-C₄-Alkyl darstellt.

26. Komplexe gemäss Anspruch 25, **dadurch gekennzeichnet, dass** R₁ für Methyl oder Ethyl steht.

27. Komplexe gemäss Anspruch 19, **dadurch gekennzeichnet, dass** R₁ Phenyl oder mit 1 oder 2 C₁-C₄-Alkyl oder C₁-C₄-Alkoxy substituiertes Phenyl darstellt.

28. Komplexe gemäss Anspruch 27, **dadurch gekennzeichnet, dass** R₂ und R₃ C₁-C₈-Alkyl darstellen.

29. Komplexe gemäss Anspruch 27, **dadurch gekennzeichnet, dass** R₂ und R₃ gleich sind und für i-Propyl oder t-Butyl stehen.

30. Komplexe gemäss Anspruch 27, **dadurch gekennzeichnet, dass** R₁₀ und R₁₁ gleich sind und für i-Propyl oder t-Butyl stehen.

31. Komplexe gemäss Anspruch 19, **dadurch gekennzeichnet, dass** R₂, R₃, R₁₀ und R₁₁ als Cycloalkyl 5 bis 8 Ring-C-Atome enthält.

32. Komplexe gemäss Anspruch 31, **dadurch gekennzeichnet, dass** R₂, R₃, R₁₀ und R₁₁ als Cycloalkyl Cyclopentyl oder Cyclohexyl darstellt.

33. Komplexe gemäss Anspruch 19, **dadurch gekennzeichnet, dass** R₂, R₃, R₁₀ und R₁₁ als substituiertes Phenyl 2-Methyl-, 3-Methyl-, 4-Methyl-, 2- oder 4-Ethyl-, 2- oder 4-i-Propyl-, 2- oder 4-t-Butyl-, 2-Methoxy-, 3-Methoxy-, 4-Methoxy-, 2- oder 4-Ethoxy-, 4-Trimethylsilyl-, 2- oder 4-Fluor-, 2,4-Difluor-, 2- oder 4-Chlor-, 2,4-Dichlor-, 2,4-Dimethyl-, 3,5-Dimethyl-, 2-Methoxy-4-methyl-, 3,5-Dimethyl-4-methoxy-, 3,5-Dimethyl-4-(dimethylamino)-, 2- oder 4-Amino-, 2- oder 4-Methylamino-, 2- oder 4-(Dimethylamnio)-, 2-oder 4-SO₃H-, 2- oder 4-SO₃Na-, 2- oder 4-[^{⊕}NH₃Cl^{⊖}]-, 2,4,6-Trimethylphen-1-yl oder 3,4,5-Trimethylphen-1-yl darstellen.

34. Komplexe gemäss Anspruch 19, **dadurch gekennzeichnet, dass** R₂ und R₃ gleich sind und Cyclohexyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl und 3,5-Dimethyl-4-methoxyphen-1-yl bedeuten.

35. Komplexe gemäss Anspruch 19, **dadurch gekennzeichnet, dass** R₂ und R₃ verschieden sind und R₂ Phenyl und R₃ Cyclohexyl, 2- oder 4-Methylphen-1-yl, 2- oder 4-Methoxyphen-1-yl, 4-(Dimethylamino)phen-1-yl, 3,5-Dimethyl-4-(dimethylamino)phen-1-yl, 3,5-Dimethyl-4-methoxyphen-1-yl oder 4-t-Butyl-phen-1-yl bedeuten.

36. Komplexe gemäss Anspruch 19, **dadurch gekennzeichnet, dass** R₂ und R₃ gleiche Reste darstellen und Cyclohexyl oder Phenyl bedeuten.

37. Komplexe gemäss Anspruch 19, **dadurch gekennzeichnet, dass** R₁ Methyl und R₂ und R₃ je Cyclohexyl oder Phenyl bedeuten.

38. Komplexe gemäss Anspruch 19, **dadurch gekennzeichnet, dass** R₁₀ und R₁₁ gleich sind und Cyclohexyl, t-Butyl, 2- oder 4-Methylphen-1-yl, 2- oder 4-Methoxyphen-1-yl, 2- oder 4-(Dimethylamino)phen-1-yl, 3,5-Dimethyl-4-(dimethylamino)phen-1-yl, 3,5-Dimethylphen-1-yl oder 3,5-Dimethyl-4-methoxyphen-1-yl bedeuten.

39. Verfahren zur asymmetrischen Hydrierung von Verbindungen mit Kohlenstoff- oder Kohle stoff-/Heteroatomdoppelbindungen unter homogenen Reaktionsbedingungen, **dadurch gekennzeichnet, dass** man die Verbindungen bei einer Temperatur von -20 bis 80 °C und einem Wasse stoffdruck von 10⁴ bis 10⁷ Pa in Gegenwart katalytischer Mengen eines Komplexes der Formel V oder VI gemäss Anspruch 19 hydriert.

40. Verfahren gemäss Anspruch 39, **dadurch gekennzeichnet, dass** der Wasserstoffdruck 10⁵ bis 6·10⁶ Pa beträgt.

41. Verfahren gemäss Anspruch 39, **dadurch gekennzeichnet, dass** die Temperatur -10 bis 50 °C beträgt.

42. Verfahren gemäss Anspruch 39, **dadurch gekennzeichnet, dass** die Menge des Katalysators so gewählt wird, dass das Molverhältnis der zu hydrierenden Verbindung (Substrat) zu dem Komplex der Formeln V oder VI 10000 bis 20 beträgt.

43. Verfahren gemäss Anspruch 39, **dadurch gekennzeichnet, dass** die Reaktion in Gegenwart eines Lösungsmittels durchgeführt wird.

44. Verfahren gemäss Anspruch 39, **dadurch gekennzeichnet, dass** das Lösungsmittel Methanol oder eine Mischung von Methanol mit Benzol oder Toluol ist.

45. Verwendung der Komplexe der Formeln V und VI gemäss Anspruch 19 als homogene Katalysatoren zur asymmetrischen Hydrierung von prochiralen Verbindungen mit Kohlenstoffoder Kohlenstoff-/Heteroatomdoppelbindungen.

46. Verwendung gemäss Anspruch 45 zur Hydrierung von unsymmetrischen Kohlenstoffdoppelbindungen, Ketiminen und Ketonen.

47. Verwendung gemäss Anspruch 45 zur Hydrierung von unsymmetrischen Kohlenstoffdoppelbindungen mit den Rhodium-Komplexen der Formeln V und VI.

## Claims

1. A compound of formula I 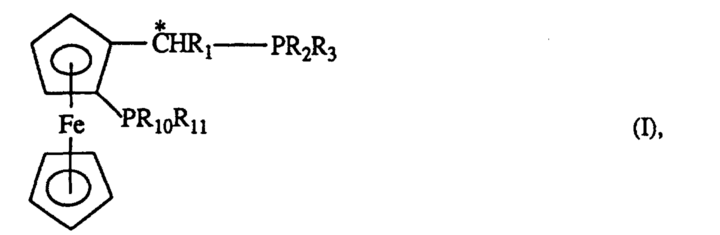 wherein R₁ is C₁-C₈alkyl, phenyl or phenyl which is substituted by 1 to 3 C₁-C₄alkyl or C₁-C₄alkoxy groups; R₂ and R₃ are each independently of the other C₁-C₁₂alkyl, C₅-C₁₂cycloalkyl, phenyl, C₁-C₄alkyl- or C₁-C₄alkoxy-substituted C₅-C₁₂cycloalkyl, or phenyl which is substituted by one to three identical or different members selected from the group consisting of C₁-C₄alkyl, C₁-C₄alkoxy, -SiR₄R₅R₆, halogen, -SO₃M, -CO₂M, -PO₃M, -NR₇R₈ and -[^{⊕}NR₇R₈R₉]X^{⊖}; or the group -PR₂R₃ is a radical of formula II 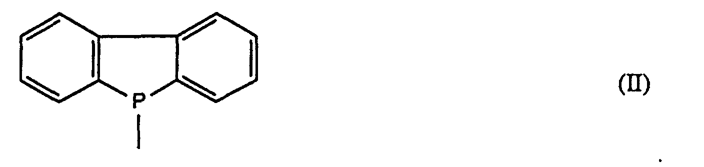 and R₄, R₅ and R₆ are each independently of one another C₁-C₁₂alkyl or phenyl, R₇ and R₈ are H, C₁-C₁₂alkyl, phenyl or R₇ and R₈, taken together, are tetramethylene, pentamethylene or 3-oxa-1,5-pentylene, R₉ is H or C₁-C₄alkyl, R₁₀ and R₁₁ are identical and are C₁-C₁₂alkyl, C₅-C₁₂cycloalkyl, C₁-C₄alkyl- or C₁-C₄alkoxy-substituted C₅-C₁₂cycloalkyl or phenyl which is substituted by 1 to 3 identical or different members selected from the group consisting of C₁-C₄alkyl, C₁-C₄alkoxy, -SiR₄R₅R₆, halogen, -SO₃M, -CO₂M, -PO₃M, -NR₇R₈ or -[^{⊕}NR₇R₈R₉]X^{⊖}; or R₁₀ and R₁₁ are different and are C₁-C₁₂alkyl, C₅-C₁₂cycloalkyl, C₁-C₄alkyl- or C₁-C₄alkoxy-substituted C₅-C₁₂cycloalkyl, phenyl or phenyl which is substituted by 1 to 3 identical or different members selected from the group consisting of C₁-C₄alkyl, C₁-C₄alkoxy, -SiR₄R₅R₆, halogen, -SO₃M, -CO₂M, -PO₃M₂, -NR₇R₈ or -[^{⊕}NR₇R₈R₉]X^{⊖}; or the group -PR₁₀R₁₁ is a radical of formula II 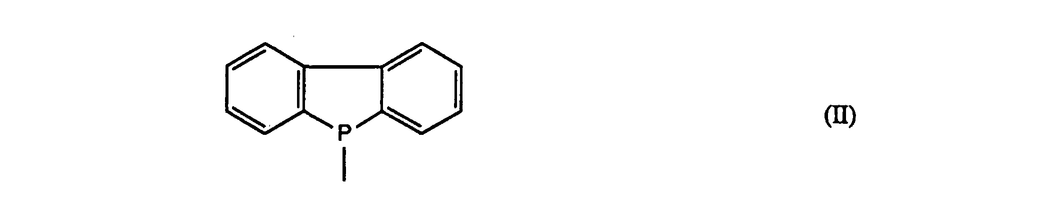 M is H or an alkali metal, X^{⊖} is the anion of a monobasic acid, and * is a stereogenic carbon atom, in the form of the racemates or diastereoisomers thereof or of a mixture of diastereoisomers.

2. A compound of formula I according to claim 1, wherein R₁ as alkyl is linear.

3. A compound of formula I according to claim 2, wherein R₁ is C₁-C₄alkyl.

4. A compound of formula I according to claim 3, wherein R₁ is methyl or ethyl.

5. A compound of formula I according to claim 1, wherein R₁ is phenyl or phenyl which is substituted by one or two C₁-C₄alkyl or C₁-C₄alkoxy groups.

6. A compound of formula I according to claim 1, wherein R₂ and R₃ are C₁-C₈alkyl.

7. A compound of formula I according to claim 6, wherein R₂ and R₃ are identical and are isopropyl or tert-butyl.

8. A compound of formula I according to claim 6, wherein R₁₀ and R₁₁ are identical and are isopropyl or tert-butyl.

9. A compound of formula I according to claim 1, wherein R₂, R₃, R₁₀ and R₁₁ as cycloalkyl contain 5 to 8 ring carbon atoms.

10. A compound of formula I according to claim 9, wherein R₂, R₃, R₁₀ and R₁₁ as cycloalkyl are cyclopentyl or cyclohexyl.

11. A compound of formula I according to claim 1, wherein R₂, R₃, R₁₀ and R₁₁ as substituted phenyl are selected from the group consisting of 2-methylphen-1-yl, 3-methylphen-1-yl, 4-methylphen-1-yl, 2- or 4-ethylphen-1-yl, 2- or 4-isopropylphen-l-yl, 2- or 4-tert-butylphen-1-yl, 2-methoxyphen-1-yl, 3-methoxyphen-1-yl, 4-methoxyphen-1-yl, 2- or 4-ethoxyphen-1-yl, 4-trimethylsilylphen-1-yl, 2- or 4-fluorophen-1-yl 2,4-difluorophen-1-yl, 2- or 4-chlorophen-1-yl, 2,4-dichlorophen-1-yl, 2,4-dimethylphen-1-yl, 3,5-dimethylphen-1-yl, 2-methoxy-4-methylphen-1-yl, 3,5-dimethyl-4-methoxyphen-1-yl, 3,5-dimethyl-4-(dimethylamino)phen-1-yl, 2- or 4-aminophen-1-yl, 2- or 4-methylaminophen-1-yl, 2- or 4-(dimethylamnio)phen-1-yl, 2- or 4-SO₃H-phen-1-yl, 2- or 4-SO₃Na-phen-1-yl, 2- or 4-[^{⊕}NH₃Cl^{⊖}]phen-1-yl, 2,4,6-trimethylphen-1-yl or 3,4,5-trimethylphen-1-yl.

12. A compound of formula I according to claim 1, wherein R₂ and R₃ are identical substituents selected from the group consisting of phenyl, cyclohexyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl and 3,5-dimethyl-4-methoxyphen-1-yl.

13. A compound of formula I according to claim 1, wherein R₂ and R₃ are different and R₂ is phenyl and R₃ is selected from the group consisting of cyclohexyl, 2- or 4-methylphen-1-yl, 2- or 4-methoxyphen-1-yl, 4-(dimethylamino)phen-1-yl, 3,5-dimethyl-4-(dimethylamino)phen-1-yl, 3,5-dimethyl-4-methoxyphen-1-yl and 4-tert-butyl-phen-1-yl.

14. A compound of formula I according to claim 1, wherein R₂ and R₃ are identical substituents and are cyclohexyl or phenyl.

15. A compound of formula I according to claim 1, wherein R₁₀ and R₁₁ are identical substituents and are preferably cyclohexyl, tert-butyl, 2- or 4-methylphen-1-yl, 2- or 4-methoxyphen-1-yl, 2- or 4-(dimethylamino)phen-1-yl, 3,5-dimethyl-4-(dimethylamino)-phen-1-yl or 3,5-dimethyl-4-methoxyphen-1-yl.

16. A compound of formula I according to claim 1, wherein R₁₀ is phenyl and R₁₁ is cyclohexyl, 2- or 4-methylphen-1-yl, 2- or 4-methoxyphen-1-yl, 4-(dimethylamino)-phen-1-yl, 3,5-dimethyl-4-(dimethylamino)phen- 1-yl, 3,5-dimethyl-4-methoxyphen-1-yl or 4-tert-butyl-phen-1-yl.

17. A compound of formula I according to claim 1, wherein R₁ is methyl and R₂ and R₃ are each cyclohexyl or phenyl.

18. A process for the preparation of a compound of formula I according to claim 1, which comprises
either reacting a compound of formula III 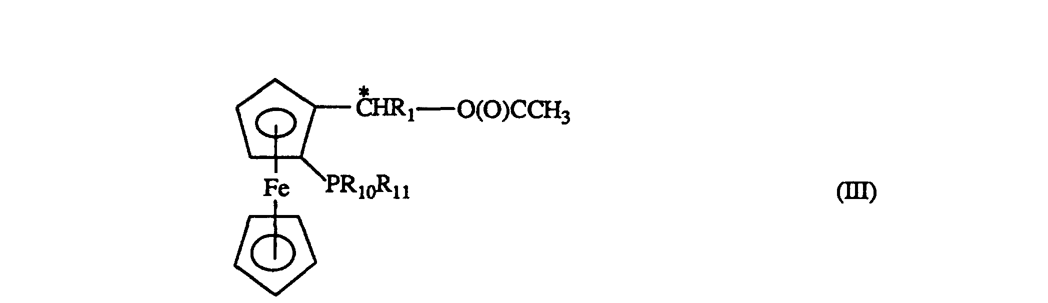 in the presence of an inert solvent, at room temperature or elevated temperature, with a phosphine of formula IV
HPR₂R₃ (IV);
or reacting a compound of formula VII 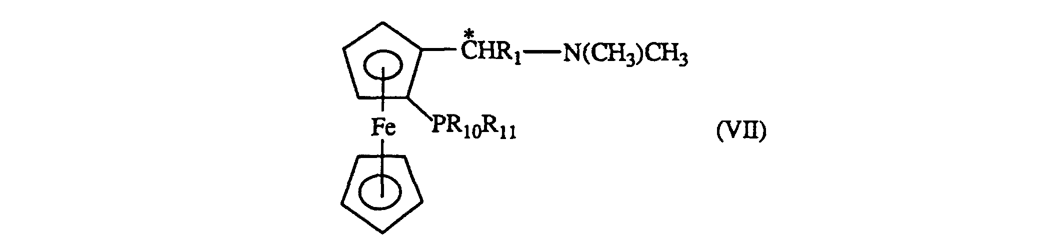 wherein R₁, R₁₀ and R₁₁ are as defined for formula I, in the presence of an inert solvent, at room temperature or elevated temperature, with a phosphine of formula IV
HPR₂R₃ (IV)
wherein R₁, R₂, R₃, R₁₀ and R₁₁ are as defined in claim 1.

19. A complex of formula V or VI
[X₁M₁YZ] (V)
[X₁M₁Y]^{⊕}A₁^{⊖} (VI)
wherein X₁ is two C₂-C₁₂olefins or a C₅-C₁₂diene, Z is Cl, Br or I, A₁^{⊖} is the anion of an oxyacid or a complex acid, M₁ is Rh or Ir, and Y is a compound of formula I as claimed in claim 1.

20. A complex according to claim 19 which is a complex of formula VI.

21. A complex according to claim 19, wherein X₁ is two ethylene, 1,5-hexadiene, 1,5-cyclooctadiene or norbornadiene.

22. A complex according to claim 19, wherein Z is Cl or Br.

23. A complex according to claim 19, wherein Z is selected from the group consisting of ClO₄^{⊖}, FSO₃^{⊖}, CH₃SO₃^{⊖}, CF₃SO₃^{⊖}, BF₄^{⊖}, PF₆^{⊖}, SbCl₆^{⊖}, AsF₆^{⊖} and SbF₆^{⊖}.

24. A complex according to claim 19, wherein R₁ as alkyl is linear.

25. A complex according to claim 24, wherein R₁ is C₁-C₄alkyl.

26. A complex according to claim 25, wherein R₁ is methyl or ethyl.

27. A complex according to claim 19, wherein R₁ is phenyl or phenyl which is substituted by one or two C₁-C₄alkyl or C₁-C₄alkoxy groups.

28. A complex according to claim 27, wherein R₂ and R₃ are C₁-C₈alkyl.

29. A complex according to claim 27, wherein R₂ and R₃ are identical and are isopropyl or tert-butyl.

30. A complex according to claim 27, wherein R₁₀ and R₁₁ are identical and are isopropyl or tert-butyl.

31. A complex according to claim 19, wherein R₂, R₃, R₁₀ and R₁₁ as cycloalkyl contain 5 to 8 ring carbon atoms.

32. A complex according to claim 31, wherein R₂, R₃, R₁₀ and R₁₁ as cycloalkyl are cyclopentyl or cyclohexyl.

33. A complex according to claim 19, wherein R₂, R₃, R₁₀ and R₁₁ as substituted phenyl are selected from the group consisting of 2-methylphen-1-yl, 3-methylphen-1-yl, 4-methylphen-1-yl, 2- or 4-ethylphen-1-yl, 2- or 4-isopropylphen-1-yl, 2- or 4-tert-butylphen-1-yl, 2-methoxyphen-1-yl, 3-methoxyphen-1-yl, 4-methoxyphen-1-yl, 2- or 4-ethoxyphen-1-yl, 4-trimethylsilylphen-1-yl, 2- or 4-fluorophen-1-yl, 2,4-difluorophen-1-yl, 2- or 4-chlorophen-1-yl, 2,4-dichlorophen-1-yl, 2,4-dimethylphen-1-yl, 3,5-dimethylphen-1-yl, 2-methoxy-4-methylphen-1-yl, 3,5-dimethyl-4-methoxyphen-1-yl, 3,5-dimethyl-4-(dimethylamino)phen-1-yl, 2- or 4-aminophen-1-yl, 2- or 4-methylaminophen-1-yl, 2- or 4-(dimethylamino)phen-1-yl, 2- or 4-SO₃H-phen-1-yl, 2- or 4-SO₃Na-phen-1-yl, 2- or 4-[^{⊕}NH₃Cl^{⊖}]phen-1-yl, 2,4,6-trimethylphen-1-yl or 3,4,5-trimethylphen-1-yl.

34. A complex according to claim 19, wherein R₂ and R₃ are identical substituents selected from the group consisting of cyclohexyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl and 3,5-dimethyl-4-methoxyphen-1-yl.

35. A complex according to claim 19, wherein R₂ and R₃ are different and R₂ is phenyl and R₃ is selected from the group consisting of cyclohexyl, 2- or 4-methylphen-1-yl, 2- or 4-methoxyphen-1-yl, 4-(dimethylamino)phen-1-yl, 3,5-dimethyl-4-(dimethylamino)phen-1-yl, 3,5-dimethyl-4-methoxyphen-1-yl and 4-tert-butylphen-1-yl.

36. A complex according to claim 19, wherein R₂ and R₃ are identical substituents and are cyclohexyl or phenyl.

37. A complex according to claim 19, wherein R₁ is methyl and R₂ and R₃ are each cyclohexyl or phenyl.

38. A complex according to claim 19, wherein R₁₀ and R₁₁ are identical substituents and are cyclohexyl, tert-butyl, 2- or 4-methylphen-1-yl, 2- or 4-methoxyphen-1-yl, 2- or 4-(dimethylamino)phen-1-yl, 3,5-dimethyl-4-(dimethylamino)phen-1-yl or 3,5-dimethyl-4-methoxyphen- 1-yl.

39. A process for the asymmetric hydrogenation of compounds containing carbon double bonds or carbon/hetero atom double bonds under homogeneous reaction conditions, which comprises hydrogenating said compounds in the temperature range from -20 to +80°C and under a hydrogen pressure of 10⁴ to 10⁷ Pa in the presence of a catalytic amount of a complex of formula V or VI as claimed in claim 19.

40. A process according to claim 39, wherein the hydrogen pressure is from 10⁵ to 6·10⁶ Pa.

41. A process according to claim 39, wherein the temperature is in the range from -10 to +50°C.

42. A process according to claim 39, wherein the amount of catalyst is chosen such that the molar ratio of compound to be hydrogenated (substrate) to complex of formula V or VI is 10 000 to 20.

43. A process according to claim 39, wherein the reaction is carried out in the presence of a solvent.

44. A process according to claim 39, wherein the solvent is methanol or a mixture of methanol and benzene or toluene.

45. Use of a complex of formula V or VI as claimed in claim 19 as homogeneous catalyst for the asymmetric hydrogenation of prochiral compounds containing carbon double bonds or carbon/hetero atom double bonds.

46. Use according to claim 45 for the hydrogenation of unsymmetric carbon double bonds, ketimines and ketones.

47. Use according to claim 45 for the hydrogenation of unsymmetric carbon double bonds with a rhodium complex of formula V or VI.

## Revendications

1. Composés de formule I, 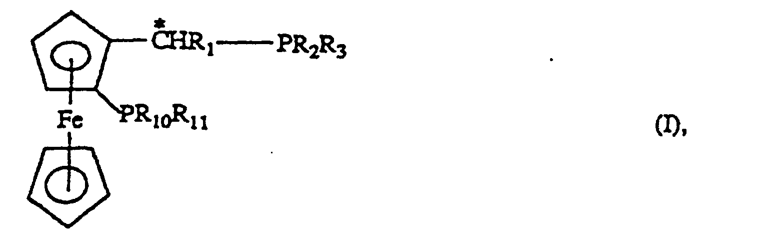 où
R₁ représente un groupe alkyle en C₁-C₈, phényle ou phényle substitué par 1 à 3 substituants alkyle en C₁-C₄ ou alkoxy en C₁-C₄ ;
R₂ et R₃ représentent, indépendamment l'un de l'autre, des groupes alkyle en C₁-C₁₂, cycloalkyle en C₅-C₁₂, phényle, cycloalkyle en C₅-C₁₂ substitué par des substituants alkyle en C₁-C₄ ou alkoxy en C₁-C₄, ou phényle substitué par un à trois substituants alkyle en C₁-C₄, alkoxy en C₁-C₄, -SiR₄R₅R₆, halogène, -SO₃M, -CO₂M, -PO₃M, -NR₇R₈ ou -[^{⊕}NR₇R₈R₉]X^{⊖} ;
ou le groupe -PR₂R₃ représente un reste de formule II 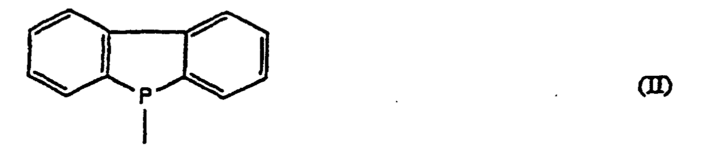 et
R₄, R₅ et R₆ représentent, indépendamment les uns des autres, des groupes alkyle en C₁-C₁₂ ou phényle,
R₇ et R₈ représentent un atome d'hydrogène, des groupes alkyle en C₁-C₁₂, phényle ou R₇ et R₈ représentent conjointement les groupes tétraméthylène, pentaméthylène ou 3-oxa-1,5-pentylène,
R₉ représente un atome d'hydrogène ou un groupe alkyle en C₁-C₄,
R₁₀ et R₁₁ sont identiques et représentent des groupes alkyle en C₁-C₁₂, cycloalkyle en C₅-C₁₂, cycloalkyle en C₅-C₁₂ substitué par des substituants alkyle en C₁-C₄ ou alkoxy en C₁-C₄, ou un groupe phényle substitué par 1 à 3 substituants alkyle en C₁-C₄, alkoxy en C₁-C₄, -SiR₄R₅R₆, halogène, -SO₃M, -CO₂M, -PO₃M, -NR₇R₈ ou -[® NR₇R₈R₉]X^{⊖} ; ou
R₁₀ et R₁₁ sont différents et représentent des groupes alkyle en C₁-C₁₂, cycloalkyle en C₅-C₁₂, cycloalkyle en C₅-C₁₂ substitué par des substituants alkyle en C₁-C₄ ou alkoxy en C₁-C₄, phényle ou phényle substitué par 1 à 3 substituants alkyle en C₁-C₄, alkoxy en C₁-C₄, -SiR₄R₅R₆, halogène, -SO₃M, -CO₂M, -PO₃M₂, -NR₇R₈ ou -[^{⊕}NR₇R₈R₉]X^{⊖} ;
ou le groupe -PR₁₀R₁₁ représente un reste de formule II 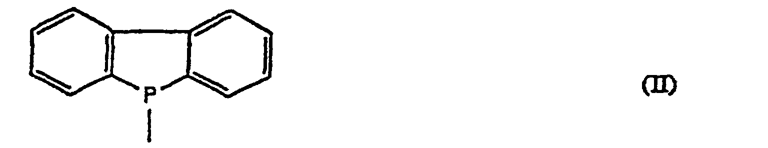
M représente un atome d'hydrogène ou un métal alcalin,
X^{⊖} représente l'anion d'un acide monofonctionnel, et
* indique un atome de carbone stéréogène, sous forme de leurs racémates et diastéréoisomères ou mélanges de diastéréoisomères.

2. Composés de formule I selon la revendication 1, **caractérisé en ce que** R₁ en tant qu'un groupe alkyle est linéaire.

3. Composés de formule I selon la revendication 2, **caractérisés en ce que** R₁ représente un groupe alkyle en C₁-C₄.

4. Composés de formule I selon la revendication 3, **caractérisés en ce que** R₁ représente un groupe méthyle ou éthyle.

5. Composés de formule I selon la revendication 1, **caractérisés en ce que** R₁ représente un groupe phényle ou phényle substitué par 1 ou 2 substituants alkyle en C₁-C₄ ou alkoxy en C₁-C₄.

6. Composés de formule I selon la revendication 1, **caractérisés en ce que** R₂ et R₃ représentent un groupe alkyle en C₁-C₈.

7. Composés de formule I selon la revendication 6, **caractérisés en ce que** R₂ et R₃ sont identiques et représentent un groupe i-propyle ou t-butyle.

8. Composés de formule I selon la revendication 6, **caractérisés en ce que** R₁₀ et R₁₁ sont identiques et représentent un groupe i-propyle ou t-butyle.

9. Composés de formule I selon la revendication 1, **caractérisés en ce que** R₂, R₃, R₁₀ et R₁₁ en tant qu'un groupe cycloalkyle présentent 5 à 8 atomes nucléaires.

10. Composés de formule I selon la revendication 9, **caractérisé en ce que** R₂, R₃, R₁₀ et R₁₁ en tant qu'un groupe cycloalkyle représentent un groupe cyclopentyle ou cyclohexyle.

11. Composés de formule I selon la revendication 1, **caractérisés en ce que** R₂, R₃, R₁₀ et R₁₁ en tant qu'un groupe phényle substitué représentent 2-méthyl-, 3-méthyl-, 4-méthyl- 2- ou 4-éthyl-, 2 ou 4-i-propyl-, 2- ou 4-t-butyl-, 2-méthoxy-, 3-méthoxy-, 4-méthoxy-, 2- ou 4-éthoxy-, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino-, 2- ou 4-(diméthylamino)-, 2- ou 4-SO₃H-, 2- ou 4-SO₃Na-, 2- ou 4-[^{⊕}NH₃Cl^{⊖}]-, 2,4,6-triméthylphén-1-yle ou 3,4,5-triméthylphén-1-yle.

12. Composés de formule I selon la revendication 1, **caractérisés en ce que** R₂ et R₃ sont identiques et représentent des groupes phényle, cyclohexyle, 2- ou 4-méthylphén-1-yle, 2- ou 4-méthoxyphén-1-yle, 2- ou 4-(diméthylamino)phén-1-yle, 3,5-diméthyl-4-(diméthylamino)phén-1-yle et 3,5-diméthyl-4-méthoxyphén-1-yle.

13. Composés de formule I selon la revendication 1, **caractérisés en ce que** R₂ et R₃ sont différents et R₂ représente un groupe phényle et R₃ un groupe cyclohexyle, 2- ou 4-méthylphén-1-yle, 2- ou 4-méthoxyphén-1-yle, 2- ou 4-(diméthylamino)phén-1-yle, 3,5-diméthyl-4-(diméthylamino)phén-1-yle, 3,5-diméthyl-4-méthoxyphén-1-yle ou 4-t-butylphén-1-yle.

14. Composés de formule I selon la revendication 1, **caractérisés en ce que** R₂ et R₃ sont des restes identiques et représentent des groupes cyclohexyle ou phényle.

15. Composés de formule I selon la revendication 1, **caractérisés en ce que** R₁₀ et R₁₁ sont des restes identiques et représentent des groupes cyclohexyle, t-butyle, 2- ou 4-méthylphén-1-yle, 2- ou 4-méthoxyphén-1-yle, 2- ou 4-(diméthylamino)phén-1-yle, 3,5-diméthyl-4-(diméthylamino)-phén-1-yle, 3,5-diméthylphén-1-yle ou 3,5-diméthyl-4-méthoxyphén-1-yle.

16. Composés de formule I selon la revendication 1, **caractérisés en ce que** R₁₀ représente un groupe phényle et R₁₁ des groupes cyclohexyle, 2 ou 4-méthylphén-1-yle, 2- ou 4-méthoxyphén-1-yle, 4-(diméthylamino)phén-1-yle, 3,5-diméthyl-4-(diméthylamino)phén-1-yle, 3,5-diméthyl-4-méthoxyphén-1-yle ou 4-t-butyl-phén-1-yle.

17. Composés de formule I selon la revendication 1, **caractérisés en ce que** R₁ représente un groupe méthyle et R₂ et R₃ représentent chacun un groupe cyclohexyle ou phényle.

18. Procédé pour la préparation de composés de formule I selon la revendication 1,
**caractérisé en ce qu'**on fait réagir
soit un composé de formule III 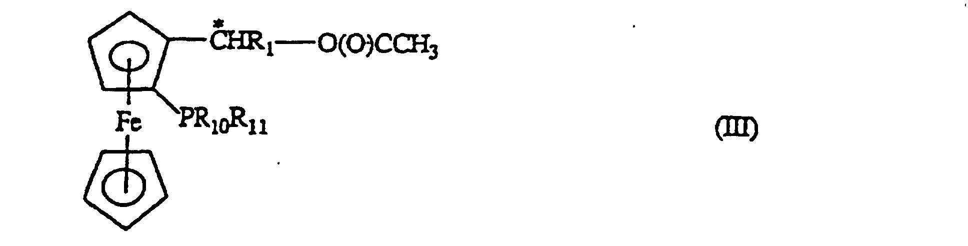 en présence d'un solvant inerte à température ambiante ou à température plus élevée sur une phosphine de formule IV
**HPR**_{**2**}**R**_{**3**} **(IV) ;**
soit un composé de formule VII 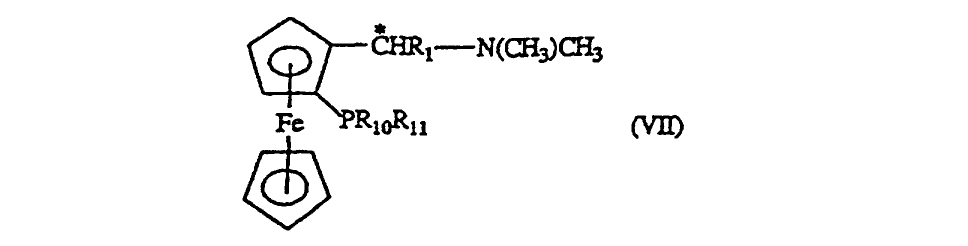 où R₁, R₁₀ et R₁₁ possèdent les significations données à la formule I, en présence d'un solvant inerte à température ambiante ou à température plus élevée sur une phosphine de formule IV
**HPR**_{**2**}**R**_{**3**} **(IV).**
R₁, R₂, R₃, R₁₀ et R₁₁ possèdent les significations données à la revendication 1.

19. Complexes de formules V et VI,
**[X**_{**1**}**M**_{**1**}**YZ] (V)**
**[X**_{**1**}**M**_{**1**}**Y]**^{**⊕**}**A**_{**1**}^{**⊖**} **(VI)**
où X₁ représente deux groupes oléfiniques en C₂-C₁₂ ou un groupe diénique en C₅-C₁₂, Z représente des atomes de Cl, de Br ou d'I, A₁ ^{⊖} représente l'anion d'un acide oxygéné ou d'un acide complexe, M₁ représente un atome de Rh ou d'Ir et Y représente un composé de formule I selon la revendication 1.

20. Complexes selon la revendication 19, **caractérisés en ce qu'**il s'agit de ceux répondant à la formule VI.

21. Complexes selon la revendication 19, caratérisés en ce que X₁ représente deux groupes éthylène, 1,5-hexadiène, 1,5-cyclooctadiène ou norbornadiène.

22. Complexes selon la revendication 19, **caractérisés en ce que** Z représente un atome de Cl ou Br.

23. Complexes selon la revendication 19, **caractérisés en ce que** Z représente des groupes ClO₄^{⊖}, FSO₃^{⊖}, CH₃SO₃^{⊖}, CF₃SO₃^{⊖}, BF₄^{⊖}, PF₆^{⊖}, SbCl₆^{⊖}, AsF₆^{⊖} et SbF₆^{⊖}.

24. Complexes selon la revendication 19, **caractérisés en ce que** R₁ en tant qu'un groupe alkyle est linéaire.

25. Complexes selon la revendication 24, **caractérisés en ce que** R₁ représente un groupe alkyle en C₁-C₄.

26. Complexes selon la revendication 25, **caractérisés en ce que** R₁ représente un groupe méthyle ou éthyle.

27. Complexes selon la revendication 19, **caractérisés en ce que** R₁ représente un groupe phényle ou phényle substitué par 1 ou 2 substituants alkyle en C₁-C₄ ou alkoxy en C₁-C₄.

28. Complexes selon la revendication 27, **caractérisés en ce que** R₂ et R₃ représentent un groupe alkyle en C₁-C₈.

29. Complexes selon la revendication 27, **caractérisés en ce que** R₂ et R₃ sont identiques et représentent un groupe i-propyle ou t-butyle.

30. Complexes selon la revendication 27, **caractérisés en ce que** R₁₀ et R₁₁ sont identiques et représentent un groupe i-propyle ou t-butyle.

31. Complexes selon la revendication 19, **caractérisés en ce que** R₂, R₃, R₁₀ et R₁₁ en tant qu'un groupe cycloalkyle présentent 5 à 8 atomes nucléaires.

32. Complexes selon la revendication 31, **caractérisés en ce que** R₂, R₃, R₁₀ et R₁₁ en tant qu'un groupe cycloalkyle représentent un groupe cyclopentyle ou cyclohexyle.

33. Complexes selon la revendication 19, **caractérisés en ce que** R₂, R₃, R₁₀ et R₁₁ en tant qu'un groupe phényle substitué représentent 2-méthyl-, 3-méthyl-, 4-méthyl- 2- ou 4-éthyl-, 2 ou 4-i-propyl-, 2- ou 4-t-butyl-, 2-méthoxy-, 3-méthoxy-, 4-méthoxy-, 2- ou 4-éthoxy, 4-triméthylsilyl-, 2- ou 4-fluoro-, 2,4-difluoro-, 2- ou 4-chloro-, 2,4-dichloro-, 2,4-diméthyl-, 3,5-diméthyl-, 2-méthoxy-4-méthyl-, 3,5-diméthyl-4-méthoxy-, 3,5-diméthyl-4-(diméthylamino)-, 2- ou 4-amino-, 2- ou 4-méthylamino-, 2- ou 4-(diméthylamino)-, 2- ou 4-SO₃H-, 2- ou 4-SO₃Na-, 2- ou 4-[^{⊕}NH₃Cl^{⊖}]-, 2,4,6-triméthylphén-1-yle ou 3,4,5-triméthylphén-1-yle.

34. Complexes selon la revendication 19, **caractérisés en ce que** R₂ et R₃ sont identiques et représentent des groupes cyclohexyle, 2- ou 4-méthylphén-1-yle, 2- ou 4-méthoxyphén-1-yle, 2- ou 4-(diméthylamino)phén-1-yle, 3,5-diméthyl-4(diméthylamino)-phén-1-yle et 3,5-diméthyl-4-méthoxy-phén-1-yle.

35. Complexes selon la revendication 19, **caractérisés en ce que** R₂ et R₃ sont différents et R₂ représente un groupe phényle et R₃ des groupes cyclohexyle, 2- ou 4-méthylphén-1-yle, 2- ou 4-méthoxyphén-1-yle, 4-(diméthylamino)phén-1-yle, 3,5-diméthyl-4-(diméthylamino)phén-1-yle, 3,5-diméthyl-4-méthoxyphén-1-yle ou 4-t-butylphén-yle.

36. Complexes selon la revendication 19, **caractérisés en ce que** R₂ et R₃ représentent chacun un groupe cyclohexyle ou phényle.

37. Complexes selon la revendication 19, **caractérisés en ce que** R₁ représente un groupe méthyle et R₂ et R₃ représentent chacun un groupe cyclohexyle ou phényle.

38. Complexes selon la revendication 19, **caractérisés en ce que** R₁₀ et R₁₁ sont des restes identiques et représentent des groupes cyclohexyle, t-butyle, 2- ou 4-méthylphén-1-yle, 2- ou 4-méthoxyphén-1-yle, 2- ou 4-(diméthylamino)phén-1-yle, 3,5-diméthyl-4-(diméthylamino)-phén-1-yle, 3,5-diméthylphén-1-yle ou 3,5-diméthyl-4-méthoxyphén-1-yle.

39. Procédé pour l'hydrogénation asymétrique de composés avec des doubles liaisons carbonées ou carbone/hétéroatome dans des conditions homogènes, **caractérisé en ce qu'**on hydrogène les composés à une température de -20 à 80°C et une pression d'hydrogène de 10⁴ à 10⁷ Pa en présence de quantités catalytiques d'un complexe de formules V ou VI selon la revendication 19.

40. Procédé selon la revendication 39, **caractérisé en ce que** la pression d'hydrogène est de 10⁵ à 6.10⁶ Pa.

41. Procédé selon la revendication 39, **caractérisé en ce que** la température va de -10 à 50°C.

42. Procédé selon la revendication 39, **caractérisé en ce que** la quantité du catalyseur est choisie de façon telle que le rapport molaire du composé à hydrogéner (substrat) au complexe de formules V ou VI soit de 10000 à 20.

43. Procédé selon la revendication 39, **caractérisé en ce qu'**on met en oeuvre la réaction en présence d'un solvant.

44. Procédé selon la revendication 39, **caractérisé en ce que** le solvant est le méthanol ou un mélange de méthanol avec du benzène ou du toluène.

45. Utilisation des complexes de formules V et VI selon la revendication 19 en tant que catalyseurs homogènes pour l'hydrogénation asymétrique de composés prochiraux avec des doubles liaisons carbonées ou carbone/hétéroatome.

46. Utilisation selon la revendication 45 pour l'hydrogénation de doubles liaisons carbonées, de cétimines et de cétones asymétriques.

47. Utilisation selon la revendication 45 pour l'hydrogénation de doubles liaisons carbonées asymétriques par des complexes de rhodium de formules V et VI.
